# EUROPEAN PATENT SPECIFICATION

(11) **EP 1 757 580 B1**
(45) Date of publication and mention of the grant of the patent: **11.06.2008**
(21) Application number: 05018481.1
(22) Date of filing: 25.08.2005
(51) Int. Cl.: C07C 319/20, C07C 319/12, C07C 41/32, C07C 29/44, C07C 321/20, C07C 321/10, C07D 305/06

(54) **Process for producing norbornene derivatives**
Verfahren zur Herstellung von Norbornenderivaten
Procédé de préparation de dérivés de norbornène

(43) Date of publication of application: 28.02.2007
(73) Proprietor: DAINIPPON INK AND CHEMICALS, INC., Itabashi-ku Tokyo (JP)
(72) Inventor: Lachowicz, Artur c/o DIC Berlin GmbH, 13403, Berlin (DE); Gaudl, Kai-Uwe c/o DIC Berlin GmbH, 13403, Berlin (DE); Takagaki, Hidetsugu c/o Dainippon Ink & Chemicals, Inc., 631, Sakado Sakura, Chiba 285-8668 (JP); Hayakawa, Hitoshi c/o Dainippon Ink & Chemicals, Inc., 631, Sakado Sakura, Chiba 285-8668 (JP)
(74) Representative: Albrecht, Thomas

(56) References cited:
- DE-A1- 2 432 630
- FR-A- 2 277 076
- US-A- 3 187 018
- US-A- 4 233 432
- US-A- 4 248 997
- PATENT ABSTRACTS OF JAPAN vol. 2003, no. 06, 3 June 2003 (2003-06-03) & JP 2003 055284 A (NIPPON PETROCHEMICALS CO LTD), 26 February 2003 (2003-02-26)

## Description

### BACKGROUND OF THE INVENTION

### FIELD OF THE INVENTION

This invention relates to a process for producing alkyl norbornene derivatives which are useful for a radically curable compound or a precursor of epoxy compounds.

### DESCRIPTION OF RELATED ART

Norbornene derivatives are widely used in the field of unsaturated polyesters as an additive to increase the compatibility of the unsaturated polyesters with styrene and to improve rigidity and toughness, as disclosed in US 4233432. The norbornene derivatives can also be used as intermediates to make cycloaliphatic epoxides, as shown in US 3187018. In particular, functionalized norbornenes and compounds having plural norbornene structures therein are useful as intermediates or as additives due to their superior compatibility and mechanical properties, which are mentioned above.

Typically, these norbornene derivatives are made by Diels-Alder reaction of cyclopentadiene and unsaturated compounds. Therefore, the functionalized norbornenes also can be made from functionalized unsaturated compounds and cyclopentadiene. For example, norbornene carbaldehyde may be prepared from cyclopentadiene and acrolein, norbornene carboxylic acid butylester is made from cyclopentadiene and butyl acrylate, carbic anhydride is made from cyclopentadiene and maleic anhydride.

In order to get high conversion in the Diels-Alder reaction, the functionalized unsaturated compound, the so called "dienophile" must be electron-poor and contains electron-attracting groups such as aldehydes, esters, ketones or anhydrides.

In case of other unsaturated "dienophile" compounds, which do not contain such strongly electron-attracting groups, the conversion of the Diels-Alder reaction was often low. In order to increase the yield, higher reaction temperatures were applied. For example, allylethers or allylalcohol react with cyclopentadiene at elevated temperatures. At the reaction temperature, the cyclopentadiene dimer is split into cyclopentadiene and reacts in situ with the allylether or allylalcohol. Allylethers are also commercially available as multifunctional materials, such trimethylolpropane mono-, di- and tri-allylether and represent therefore important precursors for multifunctional norbornenes.

However, due to the high reaction temperatures and prolonged reaction times, which are required for the conversion of allylethers, also higher amounts of oligomers of cyclopentadiene are formed, such as cyclopentadiene trimer and cyclopentadiene tetramer. For example, JP 2003055280 and JP 2003055284 describe the reaction of the cyclopentadiene dimer with allyl alcohols, yielding by-products, such as the trimer and tetramer of cyclopentadiene.

In general, the trimer, tetramer and oligomers of cyclopentadiene are difficult to separate due to the high boiling points. Often, time consuming and complicated separation procedures were required. Some by-products were hardly soluble and often caused turbidity, precipitations and smell. The problems got more severe, if multifunctional norbornene derivatives are made from multifunctional allylethers, which require a high concentration of cyclopentadiene in the batch, in order to convert all allylether functions.

Therefore, a process for the preparation of norbornene derivatives had been desired, which can suppress the yield of the trimer, tetramer and oligomers of cyclopentadiene and gives the objective compound in higher purity.

### SUMMARY OF THE INVENTION

It is accordingly an object of the invention to provide a process for producing a norbornene derivative, which can suppress the yield of the trimer, tetramer, and oligomers of cyclopentadiene, thereby can give a norbornene derivative in high yield as well as in high purity.

Unexpectedly, we have now found that the problem can be solved by reacting an allyl compound with an alkyl-cyclopentadiene dimer, and thereby the generation of the trimer, tetramer, and oligomers of cyclopentadiene can be easily suppressed.

Accordingly, the present invention provides a process for producing a norbornene derivative comprising reacting an allyl compound (A) having an allyl group represented by the following formula:

R₁-CH=CH-CH₂- (1)

wherein R₁ represents a hydrogen atom, an alkyl group, a hydroxy alkyl group, or an alkoxy alkyl group, with alkyl-cyclopentadiene dimer (B).

According to the present invention, the generation of the trimer, tetramer, and oligomers of cyclodiene can be easily suppressed compared to reactions with conventional cyclopentadiene dimer even under similar conditions.

### DETAILED DESCRIPTION OF THE PREFERRED EMBODIMENTS

As mentioned above, the process of the present invention comprises reacting an allyl compound (A) with alkyl-cyclopentadiene dimer (B).

The allyl compound (A) is characterised in having an allyl group represented by the following formula:

R₁-CH=CH-CH₂- (1)

wherein R₁ represents a hydrogen atom, an alkyl group, a hydroxy alkyl group, or an alkoxy alkyl group.

The term "alkyl" represents a hydrocarbon group having 1 to 4 carbon atoms. Thus, the alkyl group in the formula (1) includes C1 to C4 alkyl such as methyl, ethyl, propyl, or t-butyl. The hydroxy alkyl group in the formula (1) includes hydroxy methyl, hydroxy ethyl or hydroxy propyl. The alkoxy alkyl group in the formula (1) includes methoxy methyl, ethoxy methyl, or methoxy propyl.

Preferred examples of the allyl compound (A) include, for example,
a strong electron attracting group containing allyl compound such as allyl chloride, allyl fluoride, and allyl cyanide,
a weak electron attracting group containing mono-allyl compound such as allylalcohol, allyltiol, 2-butene-1,4-diol, allyloxymethyl-3-ethyloxetanylether, glycidyl allylether, pentaerythritol monoallylether, polyethyleneglycol monoallylether, trimethylol propane monoallylether,
a weak electron attracting group containing di-allyl compound such as diallylether, polyethyleneglycol diallylether, trimethylol propane diallylether, pentaerythritol diallylether, butane-1,4-diallylether,
a weak electron attracting group containing tri-allyl compound such as trimethylol propane triallylether, pentaerythritol triallylether, cyanuric acid triallylether, and
a weak electron attracting group containing mono-allyl compound such as pentaerythritol tetraallylether.

As shown above, the allyl compound (A) used in the present invention may have at least one allyl group per its molecule structure. However, in the case of selecting compounds having a plurality of allyl groups such as di-, tri-, tetra-allyl compounds, the abovementioned technical effect of the invention is remarkably enhanced. That is, in general, the compounds having a plurality of allyl groups require relatively higher temperatures for the reaction with the alkylcyclopentadiene dimer (B), thereby, oligomerization of the cyclopentadiene is inevitable.

On the contrary, according to the present invention, even if selecting higher reaction temperatures, oligomerization of cyclopentadiene can be effectively suppressed.

From this point of view, among these compounds, the di-, tri- and tetra-allyl compounds are preferable.

Furthermore, as to the allyl compound (A), it is also surprising that the reaction can be carried out even when the allyl group is bonding to a weak electron-attracting group. Namely, such a compound having only a weak electron attracting group which bonds to the allyl group normally requires higher reaction temperatures in order to enhance its reactivity. However, as mentioned above, even if adopting higher temperature conditions, oligomerization can be effectively prevented according to the present invention. From this aspect, as a compound (A), the compounds represented by the following formula (2) are preferable. 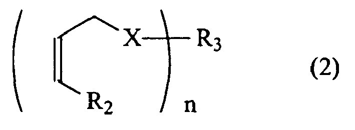 wherein R₂ represents a hydrogen atom, an alkyl group, a hydroxy alkyl group, or an alkoxy alkyl group, X denotes an oxygen atom or a sulfur atom, R₃ denotes alkyl or polyvalent alkylene, and n means an integer of 1-4.

Examples of the compound represented by the formula (2) include said weak electron attracting group containing mono-, di-, tri- and tetra-allyl compounds having an oxygen atom or a sulfur atom as the substituent X in the formula (2).

Accordingly, as regards the technical effects of the present invention, the weak electron attracting group containing di-, tri- and tetra-allyl compounds having an oxygen atom or a sulfur atom as the substituent X in the formula (2) are particularly preferable.

The alkyl-cyclopentadiene dimer (B) used in the present invention can be illustrated by the following formula (3), 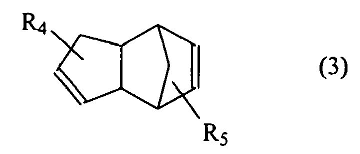 wherein R₄ and R₅ denote C1 to C4 alkyl, such as methyl, ethyl, propyl and t-butyl. Preferred among them is methyl-cyclopentadiene from the viewpoint of its superior effect of the present invention.

Surprisingly, by using the alkylcyclopentadiene dimer instead of the cyclopentadiene dimer, the formation of the methylcyclopentadiene trimer and the tetramer is largely suppressed or does not occur at all as mentioned above. Therefore, the use of the methylcyclopentadiene dimer, for example, allows the preparation of norbornene products with higher purity and less side-products.

The reaction of the allyl compound (A) with the alkyl-cyclopentadiene dimer (B), when using the compounds represented by the formula (2) as the compound (A) and the methyl-cyclopentadiene dimer as the alkyl-cyclopentadiene dimer (B), can be illustrated as follows: 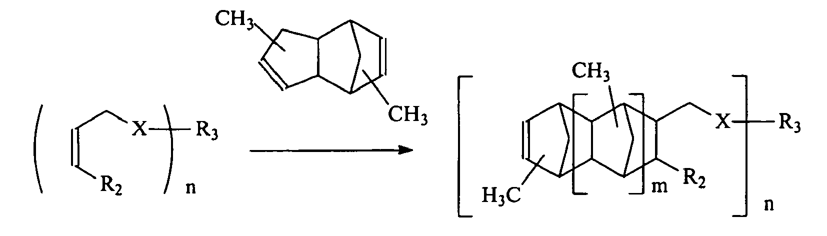 wherein X, R₂, R₃ and n are as defined in formula (2), and m means an integer 0 or 1.

The process of the present invention may be carried out as follows.

Firstly, the alkyl cyclopentadiene dimer (B) may be fed to the allyl compound (A) at room temperature or at the reaction temperature or the allyl compound (A) may be fed to the methylcyclopentadiene dimer at the temperature as stirring them.

The reaction temperatures are selected from the range of 130 to 300°C, in particular, when using the compound (B) represented by the formula (2) or the compound (B) having a plurality of allyl groups, the reaction temperatures may be raised in order to enhance their reactivity. On the other hand, when the temperature is too high, oligomerization of alkyl cyclopentadiene is easily caused. Therefore, preferred reaction temperatures are from 150 to 200°C.

Usually, the reaction is conducted under atmospheric pressure. However, in some cases, especially if the allyl-component exhibits a boiling point below 150°C or if the compound (B) shows low reactivity, the reaction is preferably performed under pressure in the range of 1.1×10⁵ Pa to 1×10⁷ Pa.

Although there are not particular restrictions on the equivalent ratios of the allyl group (a) of the allyl compound (A) and the diene group (b) of the alkylcyclopentadiene dimer (B), when the allyl compound (A) has a plurality of allyl groups, it can be selected in the range from (a):(b) = 1: 1 to 1:1.3. On the other hand, in case of using a mono-functional compound as the compound (A), by selecting the equivalent ratio within the range of (a):(b) = 1.1:1 to 2: 1, the formation of oligomers of alkyl cyclodiene can be suppressed.

In the process of the present invention, a solvent is not necessarily required, if used, inert solvents such as xylene or decaline are preferred. The process may be run batch-wise or in continuous fashion, for example in a tubular reactor.

In case that during the reaction time the colour of the reaction mixture gets darker, the use of an inert gas such as nitrogen, is preferable.

When the reaction subsides, residual starting materials are removed by distillation and recovered for the next batch.

The products obtained according to the process of the invention may be purified according to the customary techniques used by persons skilled in the art.

Thus obtained norbornene derivatives have various chemical structures depending on their starting materials as follows:
a) Examples of compounds having a strong electron attracting group: 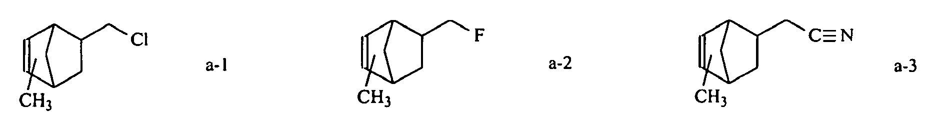
b) Examples of compounds having mono-norbornene structure: 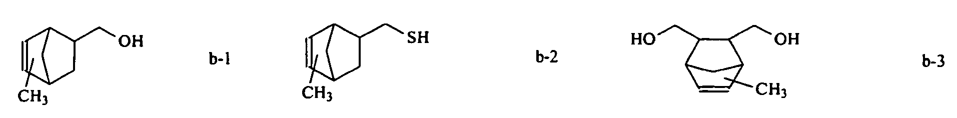 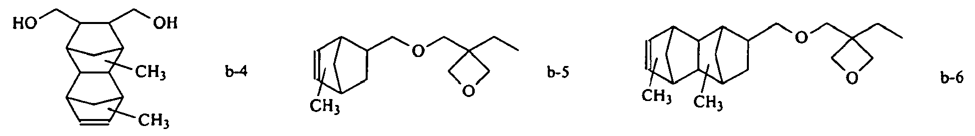 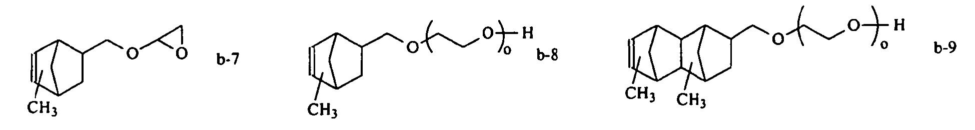 wherein o means an integer of 1 to 7, p means an integer of 1 to 7.
c) Examples of compounds having di-norbornene structure: 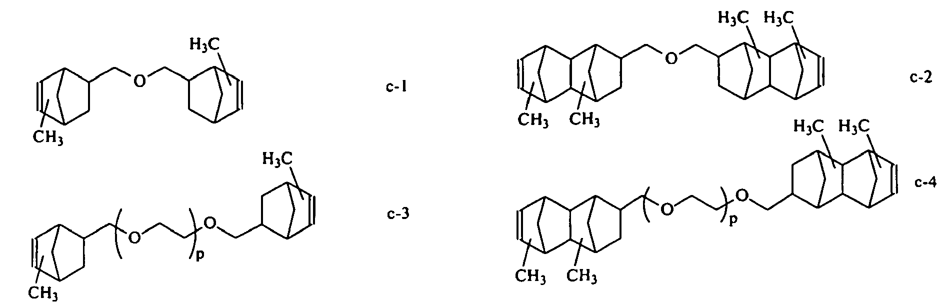 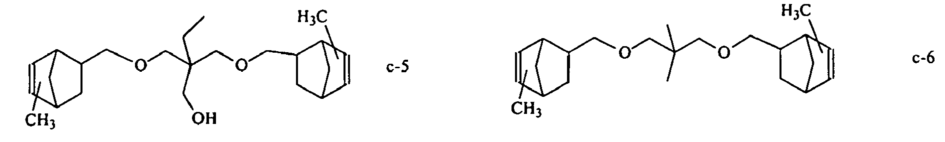 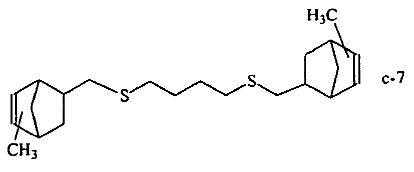
d) Examples of compounds having tri-norbornene structure: 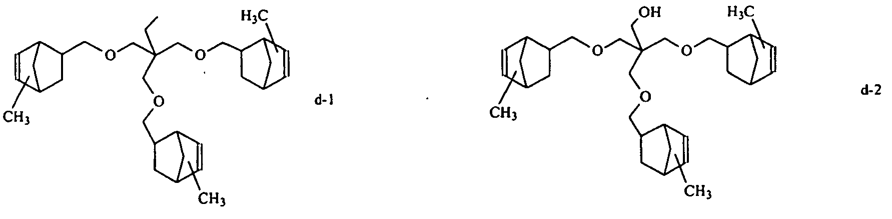 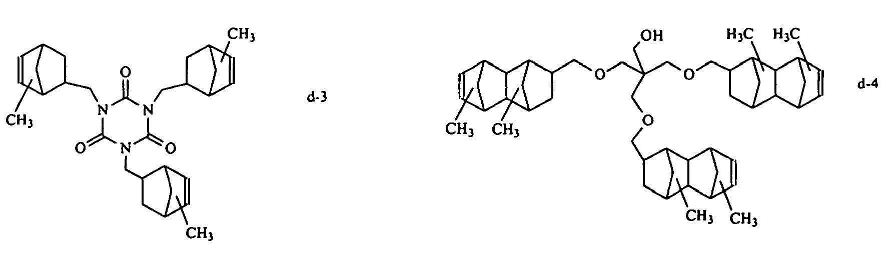
e) Examples of compounds having tetra-norbornene structure:

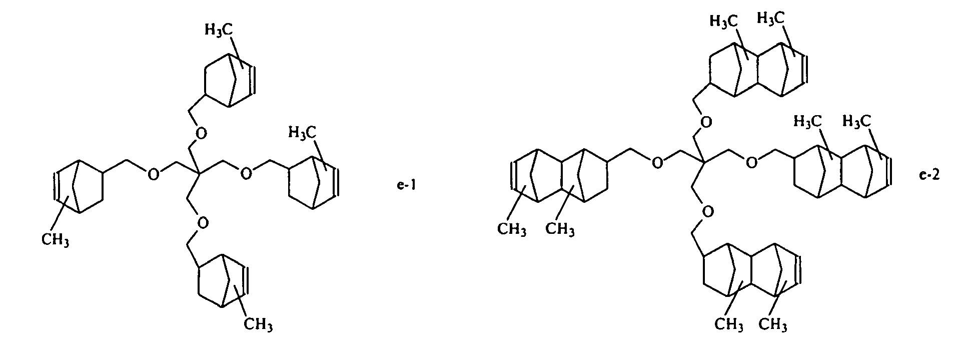

Here, the position of the methyl group is not exactly defined as shown by the structures, because the reaction creates isomers, having the methyl group in different positions of the norbornene system.

Among them, as mentioned above, when using an allyl compound having only a weak electron attracting group as an allyl compound (A), the effect of the present invention can be prominent, therefore, the norbornene derivatives having the following general formula (4) are preferred.

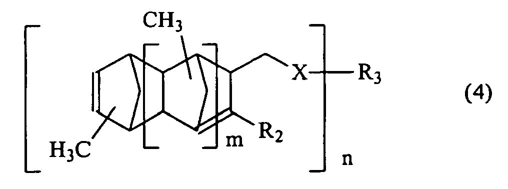

Here, wherein X, R₂ and R₃ are as defined in formula (2),n means an integer of 1 to 4, m means an integer of 0 or 1.

Specific examples of the compound of formula (4) include the above illustrated compounds (b) to (e).

In particular, preferred among these compounds are compounds having a plurality of norbornene structures per one molecular such as the above illustrated the compounds (c) to (e) due to their usefulness as a crosslinking agent or excellent curability after their epoxidation.

The compounds prepared according to this invention can be used as additives for unsaturated polyesters in order to improve the compatibility of the unsaturated polyesters with styrene and to increase the rigidity and toughness or as intermediates for making cycloaliphatic epoxides.

### Examples

### Example 1

In a 11 four-necked flask, fitted with a thermometer, mechanical stirrer, reflux condenser and nitrogen inlet tube, is placed 584.0g (4.0 moles) of 3-allyloxymethyl-3-ethyloxetanylether and 320.5g (2.0 moles) of methylcyclopentadiene dimer. The mixture is purged with nitrogen and heated to 170°C. At a conversion of 90% of the 3-allyloxy-methyl-3-ethyloxetanylether, residual starting materials were removed by distillation under light vacuum (20-50 mbar). Then, the reaction mixture was allowed to cool down to room temperature and was analysed standardized by GC-MS:

| | |
|---|---|
| Product 1: | 95.2% |
| 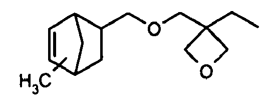 | |
| Product 2: | 4.0% |
| 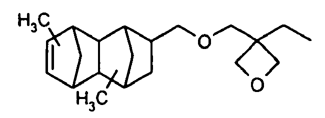 | |
| Side-product: | 0.8% |
| 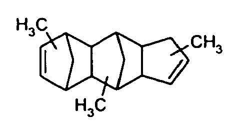 | |

### Example 2 (comparative example)

In a 11 four-necked flask, fitted with a thermometer, mechanical stirrer, reflux condenser and nitrogen inlet tube, is placed 584.0g (4.0 moles) of 3-allyloxymethyl-3-ethyloxetanylether and 264.0g (2.00 moles) of cyclopentadiene dimer. The mixture is purged with nitrogen and heated to 170°C. At a conversion of 90% of the 3-allyloxymethyl-3-ethyloxetanylether, residual starting materials were removed by distillation under light vacuum (20-50 mbar). Then, the reaction mixture was allowed to cool down to room temperature and was analysed standardized by GC-MS:

| | |
|---|---|
| Product 1: | 81.3% |
| 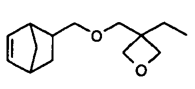 | |
| Product 2: | 10.4% |
| 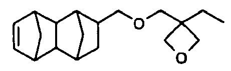 | |
| Side-product: | 8.3% |
| 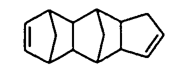 | |

### Example 3

In a 11 four-necked flask, fitted with a thermometer, mechanical stirrer, reflux condenser and nitrogen inlet tube, is placed 317.0g (1.48 moles) of trimethylol propane diallylether and 238.0g (1.48 moles) of methylcyclopentadiene dimer. The mixture is purged with nitrogen and heated to 180°C. At a conversion of 90% of the residual starting materials were removed by distillation under vacuum (0.1-1 mbar). Then, the reaction mixture was allowed to cool down to room temperature and was analysed standardized by GC-MS:

| | |
|---|---|
| Product 1: | 89.5% |
| 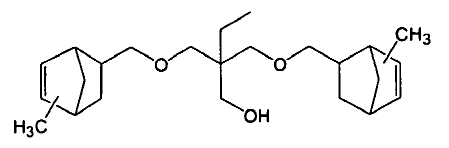 | |
| Product 2: | 6.5% |
| 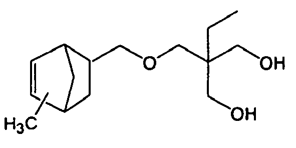 | |
| Product 3: | 3.5% |
| 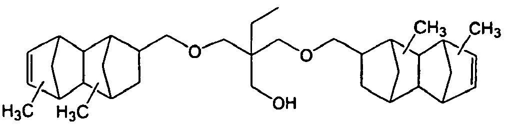 | |
| Side-product 1: | 0.5% |
| 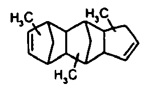 | |

### Example 4 (comparative example)

In a 11 four-necked flask, fitted with a thermometer, mechanical stirrer, reflux condenser and nitrogen inlet tube, is placed 535.0g (2.50 moles) of trimethylol propane diallylether and 330.5g (2.50 moles) of cyclopentadiene dimer. The mixture is purged with nitrogen and heated to 180°C. At a conversion of 90% of the residual starting materials were removed by distillation under vacuum (2-5 mbar). Then, the reaction mixture was allowed to cool down to room temperature and was analysed standardized by GC-MS:

| | |
|---|---|
| Product 1: | 80.5% |
| 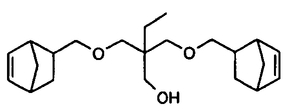 | |
| Product 2: | 6.9% |
| 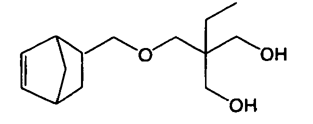 | |
| Product 3: | 4.5% |
| 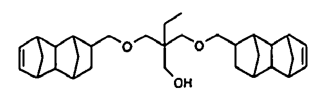 | |
| Side-product 1: | 8.1% |
| 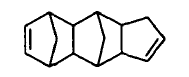 | |

### Example 5

146.3g (2.52 moles) of allylalcohol was placed in an autoclave. The autoclave was evacuated, filled with nitrogen and heated to 200°C. Then, 201.6 g (1.26 moles) of methylcyclopentadiene dimer was fed over 2 hours. After the addition was complete, the temperature was maintained at 200°C for another hour and cooled to room temperature. The reaction mixture was analysed by liquid chromatography and mass-spectroscopy.

| | |
|---|---|
| Allylalcohol | 6.6% |
| Methylcyclopentadiene dimer | 2.8% |
| Methyl-5-norbornene-2-methanol | |
| 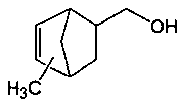 | 73.5% |
| Dimethyloctahydrodimethanonaphtalene-methanol | |
| 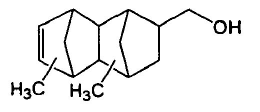 | 16.2% |
| Trimer of methylcyclopentadiene | 0.8% |
| Tetramer of methylcyclopentadiene | 0.1% |

### Example 6 (comparative example)

146.3g (2.52 moles) of allylalcohol was placed in an autoclave. The autoclave was evacuated, filled with nitrogen and heated to 200°C. Then, 166.6g (1.26 moles) of cyclopentadiene dimer was fed in small portions over 2 hours. After the addition was complete, the temperature was maintained at 200°C for another hour and cooled to room temperature. The reaction mixture was separated by liquid chromatography and analysed by mass-spectroscopy.

| | |
|---|---|
| Allylalcohol | 6.1% |
| Cyclopentadiene dimer | 1.9% |
| 5-norbornene-2-methanol | |
| 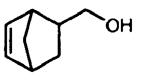 | 70.5% |
| Octahydrodimethanonaphtalene-methanol | |
| 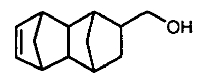 | 13.2% |
| Trimer of cyclopentadiene | 5.3% |
| Tetramer of cyclopentadiene | 3.0% |

### Example 7

100.0g (1.13 moles) of 2-butene-1,4-diol was placed in a flask, purged with nitrogen and heated to 195°C. Then, 80.0g (0.50 moles) of methylcyclopentadiene dimer was dropped to 2-butene-1,4-diol over 5 hours at 195-200°C. After the addition was complete, the temperature was maintained at 200°C for another hour and cooled to room temperature. The reaction mixture was analysed by liquid chromatography and gas-chromatography:

| | |
|---|---|
| 2-Butene-1,4-diol | 15.0% |
| Methylcyclopentadiene dimer | 2.1% |
| Methyl-5-norbornene-2,3-dimethanol | |
| 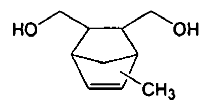 | 65.5% |
| Octahydrodimethyldimethanonaphtalene-dimethanol | |
| 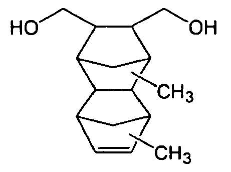 | 13.8% |
| Methylcyclopentadiene trimer | 2.3% |
| Methylcyclopentadiene tetramer | 0.3% |

### Example 8 (comparative example)

100.0g (1.13 moles) of 2-butene-1,4-diol was placed in a flask, purged with nitrogen and heated to 195°C. Then, 66.0g (0.50 moles) of cyclopentadiene dimer was dropped to 2-butene-1,4-diol over 5 hours at 195-200°C. After the addition was complete, the temperature was maintained at 200°C for another hour and cooled to room temperature. The reaction mixture was analysed by liquid chromatography and gas-chromatography:

| | |
|---|---|
| 2-Butene-1,4-diol | 16.0% |
| Cyclopentadiene dimer | 4.1% |
| 5-Norbornene-2,3-dimethanol | |
| 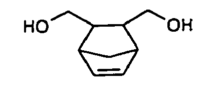 | 58.5% |
| Octahydrodimethanonaphtalene-dimethanol | |
| 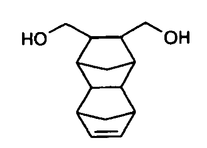 | 12.0% |
| Cyclopentadiene trimer | 7.1% |
| Cyclopentadiene tetramer | 2.3% |

## Claims

1. A process for producing a norbornene derivative comprising:
reacting an allyl compound (A) having an allyl group represented by the following formula (1),
R₁-CH=CH-CH₂- (1)
wherein R₁ represents a hydrogen atom, an alkyl group, a hydroxy alkyl group, or an alkoxy alkyl group, with alkyl-cyclopentadiene dimer (B).

2. The process for producing a norbornene derivative according to claim 1, wherein the allyl compound (A) has a plurality of allyl groups represented by the following formula (1) ,
R₁-CH=CH-CH₂- (1)
wherein R₁ represents a hydrogen atom, an alkyl group, a hydroxy alkyl group, or an alkoxy alkyl group.

3. The process for producing a norbornene derivative according to claim 1 or 2, wherein the allyl compound (A) is represented by the following formula (2), 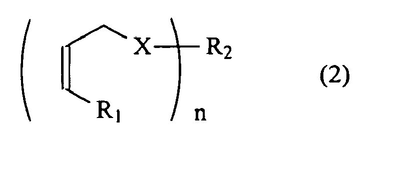 wherein R₁ represents a hydrogen atom, an alkyl group, a hydroxy alkyl group, or an alkoxy alkyl group,
X denotes an oxygen atom or a sulfur atom,
R₂ denotes alkyl or polyvalent alkylene, and
n means an integer of 1-6.

4. The process for producing a norbornene derivative according to claim 1 or 2, wherein the reaction is carried out at a temperature of 150 to 200°C.

## Patentansprüche

1. Verfahren zum Herstellen eines Norbornenderivats, umfassend:
Umsetzen einer Allylverbindung (A) mit einer Allylgruppe, dargestellt durch die folgende Formel (1),
R₁-CH=CH-CH₂- (1)
worin R₁ ein Wasserstoffatom, eine Alkylgruppe, eine Hydroxyalkylgruppe oder eine Alkoxyalkylgruppe darstellt, mit einem Alkylcyclopentadiendimer (B).

2. Verfahren zum Herstellen eines Norbornenderivats gemäß Anspruch 1, wobei die Allylverbindung (A) eine Vielzahl von Allylgruppen hat, dargestellt durch die folgende Formel (1),
R₁-CH=CH-CH₂- (1)
worin R₁ ein Wasserstoffatom, eine Alkylgruppe, eine Hydroxyalkylgruppe oder eine Alkoxyalkylgruppe darstellt.

3. Verfahren zum Herstellen eines Norbornenderivats gemäß Anspruch 1 oder 2, wobei die Allylverbindung (A) durch die folgende Formel (2) dargestellt wird, 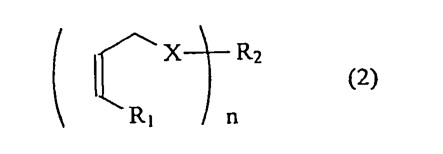 worin R₁ ein Wasserstoffatom, eine Alkylgruppe, eine Hydroxyalkylgruppe oder eine Alkoxyalkylgruppe darstellt,
X ein Sauerstoffatom oder ein Schwefelatom bezeichnet,
R₂ Alkyl oder polyvalentes Alkylen bezeichnet und
n eine ganze Zahl von 1-6 bedeutet.

4. Verfahren zum Herstellen eines Norbornenderivats gemäß Anspruch 1 oder 2, wobei die Reaktion bei einer Temperatur von 150 bis 200°C durchgeführt wird.

## Revendications

1. Procédé de préparation d'un dérivé de norbornène comprenant :
la mise en réaction d'un composé allylique (A) ayant un groupe allyle représenté par la formule (1) suivante
R₁-CH=CH-CH₂- (1)
où R₁ représente un atome d'hydrogène, un groupe alkyle, un groupe hydroxyalkyle, ou un groupe alcoxyalkyle,
avec un dimère alkyl-cyclopentadiène (B).

2. Procédé de préparation d'un dérivé de norbornène selon la revendication 1, dans lequel le composé allylique (A) a une pluralité de groupes allyles représentés par la formule (1) suivante
R₁-CH=CH-CH₂- (1)
où R₁ représente un atome d'hydrogène, un groupe alkyle, un groupe hydroxyalkyle, ou un groupe alcoxyalkyle.

3. Procédé de préparation d'un dérivé de norbornène selon la revendication 1 ou 2, dans lequel le composé allylique (A) est représenté par la formule (2) suivante 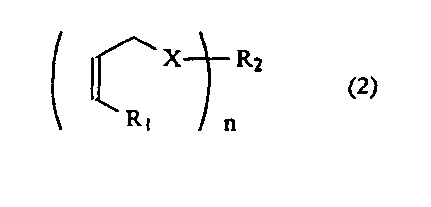 où R₁ représente un atome d'hydrogène, un groupe alkyle, un groupe hydroxyalkyle, ou un groupe alcoxyalkyle,
X représente un atome d'oxygène ou un atome de soufre,
R₂ représente un groupe alkyle ou alkylène polyvalent, et
n est un nombre entier de 1 à 6.

4. Procédé de préparation d'un dérivé de norbornène selon la revendication 1 ou 2, dans lequel la réaction est réalisée à une température de 150 à 200 °C.
